(19) Europäisches Patentamt
European Patent Office
Office européen des brevets

(11) EP 0 599 704 B1

(12) **FASCICULE DE BREVET EUROPEEN**

(45) Date de publication et mention de la délivrance du brevet:
**27.01.1999 Bulletin 1999/04**

(51) Int. Cl.[6]: **C07C 209/10**, C07C 209/62, C07C 211/59

(21) Numéro de dépôt: **93402808.5**

(22) Date de dépôt: **19.11.1993**

(54) **Voie de synthèse vers des anilines désactivées**

Syntheseweg zu desaktivierten Anilinen

Synthesis path for deactivated anilines

(84) Etats contractants désignés:
**AT BE CH DE DK ES FR GB GR IE IT LI LU NL PT SE**

(30) Priorité: **25.11.1992 FR 9214157**
**25.11.1992 FR 9214160**
**25.11.1992 FR 9214155**

(43) Date de publication de la demande:
**01.06.1994 Bulletin 1994/22**

(60) Demande divisionnaire:
**98108235.7 / 0 861 825**

(73) Titulaire: **RHODIA CHIMIE**
**92408 Courbevoie Cédex (FR)**

(72) Inventeur: **Ricca, Jean-Marc**
**F-69007 Lyon (FR)**

(74) Mandataire:
**Seugnet, Jean Louis et al**
**RHODIA SERVICES,**
**Direction de la Propriété Industrielle**
**25, quai Paul Doumer**
**92408 Courbevoie Cedex (FR)**

(56) Documents cités:
**US-A- 2 887 514**

- **SYNTHESIS Janvier 1980 , STUTTGART DE pages 39 - 41 T. WATANABE E.A. 'A Convenient Synthesis of Methylamino and Dimethylamino Substituted Aromatic Compounds'**
- **'Methoden der Organischen Chemie (Houben-Weyl) Band XI/1 Stickstoffverbindungen II' 1957 , GEORG THIEME VERLAG , STUTTGART * page 979, alinéa 11 ***
- **M. FIESER, L.F.FIESER 'Reagents for Organic Synthesis Volume 3' 1972 , WILEY-INTERSCIENCE , NEW-YORK ETC * page 239 - page 240 ***
- **CHEM. BER. (CHBEAM); 67; VOL.100 (7); PP.2131-7 'Über alpha.-halogenierte Amine. XVII. Über N-(Chloromethyl)-aniline and N-(aryl-substitutierte) Aminale'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7/3.6

## Description

La présente invention a pour objet la synthèse d'anilines désactivées. Elle concerne plus particulièrement un procédé comportant une étape de dialcoylamination au moyen d'un amide. La synthèse des anilines se fait en général au moyen des dérivés nitrés que l'on hydrogène.

Toutefois, cette technique est soumise à de nombreuses contraintes et n'est pas toujours facile à appliquer lorsque des produits sont sensibles à l'hydrogénolyse ou lorsque elle donne lieu à de nombreuses réactions parasites.

En outre, le positionnement de la fonction nitrée n'est pas toujours aisé et certaines synthèses nécessitent de nombreuses étapes.

le document US2887514 décrit une technique d'amination par l'action de formamide sur des dérivé chloré, cependant les résultats reste moyen pour les dérivé nitrés mais sont extrêmement médiocres dans le cas de dérivé non nitré.

l'article publié dans Synthesis de janvier 1980 pages 39 à 41 de T. Watanabe enseigne qu'en utilisant un amide en présence de potasse on obtient de très bon résultat pour des dérivé nitré les dérivé à noyau pyridine mais très variable pour d'autres dérivés hétérocycliques."

La présente invention vise à apporter une nouvelle voie de synthèse utilisable pour faire des anilines substituées par un groupe électroattracteur (GEA), notamment en para. Ce but, ainsi que d'autres qui apparaîtront par la suite sont atteints au moyen d'un procédé comportant une étape consistant à faire réagir un composé de formule (I)

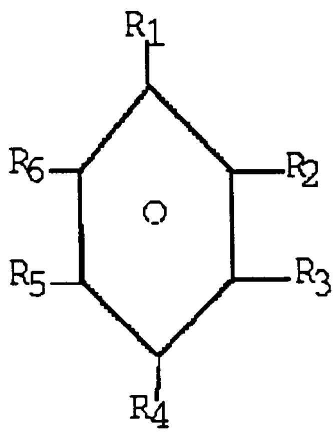

(I)

avec :

- $R_1$ choisi parmi les groupes dont les anions ($R_1^-$) constituent d'excellents groupes partants, avantageusement parmi les atomes de brome ou de chlore, de préférence ce dernier ; sont considérés comme bon groupes partant, ceux dont l'acide associé à l'anion présentent une valeur de pKa au plus égale à 1, avantageusement à 0, de préférence à -1.

- $R_2$ choisi parmi l'hydrogène, les chaînes hydrocarbonées, les halogènes, les groupes électro-attracteurs (GEA) avantageusement par effet inducteur mais non mésomère ;

- $R_3$ est indifférent mais il est avantageux qu'il soit choisi parmi l'hydrogène et les groupes au moins aussi attracteurs par effet inductif que les groupes alcoyles ;

- $R_4$ est choisi parmi l'hydrogène, les chaînes hydrocarbonées les halogènes et les groupes électro-attracteurs par effet inducteur mais non mésomère;

- $R_5$ est indifférent mais il est avantageux qu'il soit choisi parmi l'hydrogène, les groupes au moins aussi attracteurs par effet inductif que les groupes alcoyles ;

- $R_6$ choisi parmi les chaînes hydrocarbonées les halogènes les groupes électroattracteurs, avantageusement par effet inducteur mais non mésomère; avec la condition que au moins un des groupes $R_1$, $R_6$ et de préférence $R_4$ est électro-attracteur par effet inductif ;

$R_2$ et $R_3$ et/ou $R_5$ et $R_6$ pouvant former au moins un cycle, éventuellement aromatique, le cas échéant substitué; sur un dialcoylamide en présence d'une base et à une température comprise entre 150 et 300°C de préférence entre 180°C et 250°C (dans la présente description, sauf mention contraire, les zéros de position ne sont pas significatifs).

Un des éléments de surprise vient du fait que l'on ait pu trouver des conditions où la réaction fonctionne avec moins de deux groupes nitro sur un même noyau et même sans.
Avantageusement, soit du fait de leur nature soit du fait de la disposition des groupes Électroattracteurs, les anions ($R_1^-$) constituent de meilleurs groupes partants que les anions issus des radicaux $R_2$ à $R_6$.
Avantageusement, le(s) groupe(s) électroattracteur(s) (GEA) est (sont) choisi(s) parmi les groupes pseudo-halogènes et alcoxycarbonyles, halogène léger (Fluor, chlore, brome), le plus souvent chlore et avantageusement perhalogénoalcoyle, et plus avantageusement trihalogénométhyle, de préférence perfluoroalcoyle.
Les radicaux $R_3$ et $R_5$ sont avantageusement peu encombrants et de préférence des hydrogènes, des pseudo-halogènes ou des halogènes, avantageusement fluor ou chlore à condition qu'ils soient moins bon groupe partant que $R_1$.
On entend par pseudohalogène ceux des radicaux susceptibles de constituer des groupes partants anioniques dont l'acide associé à l'anion présentent une valeur de pKa au plus égale à 1, avantageusement à 0, de préférence à -1.
On entend par perhalogénoalcoyle les groupes répondant à la formule

$$R\text{-}(CX_2)_{n}\text{-} \qquad \text{(III)}$$

- → où n est au moins égale à 1 et lorsque R n'est pas électroattracteur de préférence égal à 2
- → où les X sont choisis indépendamments parmis les halogènes de préférence parmi le fluor et le chlore,
- → où Y est un groupe partant de préférence le fluor, le chlore, le brome ou l'iode, & où R est un reste hydrocarboné, alcoyle ou aryle, de C1 à C10, avantageusement de préférence de C1 à C6 de préférence de C1 à C4, de préférence un électroattracteur, avantageusement un X (halogène de préférence chlore ou fluor).

Les X sont le plus souvent identiques.

n est en général au plus égal à 10 et le plus souvent, notamment lorsque le perfluoroalcoyle est en position ortho, inférieur à 6, de préférence à 4.

Le solvant peut être tout solvant non-décomposable dans les conditions de l'expérience et inerte vis à vis des réactifs, avantageusement polaire (c'est-à-dire dont la constante diélectrique $\varepsilon$ est au moins égal à 30).
Lorsqu'il est protique, et a fortiori lorsqu'il ne l'est pas, il présente un pKa avantageusement au moins égal à celui de l'eau, à savoir 14.
Il va de soi que par solvant on entend tout mélange ou tout produit pur, susceptible d'être utilisé comme solvant.

Toutefois, on préfère travailler en utilisant comme seul solvant soit un excès de réactifs, soit un excès de substrat.
Parmi les bases utilisables, on préfère les bases anioniques y compris les carbonates.
Les bases utilisées sont avantageusement les oxydes de métaux alcalins, des amidures alcalins, des hydroxydes et des alcoolates alcalins.
Pour obtenir de bon résultat, il est souhaitable que le pKa de l'acide conjugué de la base soit au moins égal à 9, avantageusement 10, de préférence à 12.
Il est à noter que le système tolère comme solvant les dérivés hydroxylés et notamment l'eau, surtout si l'on utilise du cuivre comme catalyseur.

Alors, avantageusement, la réaction est menée en présence de cuivre, ce dernier est de préférence introduit sous forme de sel cuivreux et en quantité comprise entre 0,1% et 10% en mole par rapport au substrat
Les quantités de réactifs et catalyseurs avantageusement mises en jeu sont les suivantes en prenant comme référence la quantité de substrat égal à 1 :

Base: de 0,5 à 5 équivalents-grammes, de préférence entre 2 et 4,

Amide : de 1 à 100 équivalents-grammes, de préférence de 5 à 25.

Les amides sont de préférence les dialcoylamides des acides carboxyliques légers (au plus 10 atomes de carbone).
Il est possible d'utiliser des diamides tel que les tétraméthylurées, on peut également utiliser la tetraméthylguanidine ; les meilleurs résultats sont obtenus lorsque les dialcoylamides sont des dialcoylformamides, les radicaux alcoyles greffés sur l'azote des amides étant avantageusement légers, de manières que le nombre d'atome de carbone n'excède 6,

avantageusement 4, de préférence 3. Les dérivés méthylés sont les dérivés préférés.
La réaction a lieu en autoclave à la pression naturelle des réactifs dans les conditions expérimentales.
Quoique cela ne soit pas indispensable, il est préférable de travailler sous atmosphère inerte, par exemple sous azote ou sous argon.

L'invention présente un intérêt particulier pour obtenir des dérivés trifluorométhylés.

En effet, contre toute attente, les groupes perfluoroalcoyles, et notamment le groupe trifluorométhyle, sont stables dans les conditions opératoires de la présente invention. Selon la présente invention, il a été ainsi possible de réaliser la synthèse de paratrifluorométhyl-N,N diméthylanilines en une seule étape à partir des dérivés halogénés, et notamment chlorés, correspondants.

Les résultats sont meilleurs lorsque l'on part d'un dérivé dihalogéné ou trihalogéné, de préférence dichloré ou trichloré, en position méta et para d'une fonction électroattractrice perfluoroalcoyle, en général trifluorométhyle.
En d'autres termes la synthèse représente un intérêt particulier lorsque dans la formule (I) $R_1$est chlore ou brome de préférence chlore ; $R_2$ est fluor, chlore ou brome de préférence chlore ; $R_3$ est hydrogène, fluor, chlore ou brome de préférence chlore ; $R_4$ est perfluoroalcoyle, en général trifluorométhyle.

Toutefois les composés obtenus par cette étape de la présente invention ne sont pas tous utilisables directement ; on peut même dire que l'aniline primaire, voire même secondaire, est plus intéressante que l'aniline tertiaire correspondante.

Or la technique usuelle pour déméthyler les anilines consiste à utiliser l'acide bromhydrique (HBr) aqueux qui risque de détruire les fonctions que l'on désire présentes sur le noyau conjointement à la fonction l'aniline. Comme fonction dont la fragilité est avérée, il convient de citer les fonctions halogénées, plus souvent perhalogénées en position benzylique, c'est à dire en $\alpha$ du noyau aromatique, en particulier la fonction perfluoroalcoyle, le plus souvent trifluorométhyle.
C'est pourquoi un des buts de la présent invention est de fournir un procédé de dé alcoylation des anilines notamment celles qui sont désactivées c'est à dire dont l'acide associé présente un pKa au plus égal à 2 de préférence à 1.

Un autre but de la présente invention est de fournir un procédé du type précédent qui n'abîme pas les fonction fragiles définies ci dessus, telle que la fonction perfluoroalcoyle, en général trifluorométhyle

Un autre but de la présente invention est de fournir un procédé du type précédent qui ne touche pas les substituants ni les positions en méta de l'aniline.

Ces buts, ainsi que d'autres qui apparaîtront par la suite sont atteints par un procédé pour N déalcoyler une aniline avantageusement désactivée, mono- ou dialcoylée et il comporte l'étape d'halogénation en elle même connue au moyen d'un agent d'halogénation de type radicalaire.

Les techniques de déalcoylation au moyen d'halogènes telle que décrites dans le Houben Weil n'a guère donnée de résultat concluant sur les substrats visés par la présente invention. (se reporter à l'exemple 25)".

L'halogénation selon l'invention est menée dans des conditions réputées radicalaires, avec, de préférence, une initiation (utilisation de radiation électromagnétique de longueur d'onde convenable, de peroxydes ou équivalents).
Elle est conduite avantageusement seur les composés de formule (II) suivante :

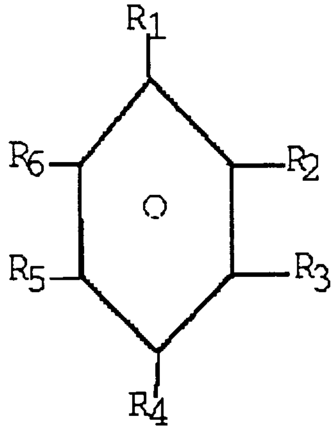

où $R_1$représente un radical mono- ou dialcoyamino- et

- $R_2$ choisi parmi l'hydrogène, les chaînes hydrocarbonées les halogènes les groupes électro-attracteurs (GEA) par effet inducteur mais non mésomère;

- $R_3$ est indifférent mais il est avantageux qu' il soit choisi parmi l'hydrogène, les groupes au moins aussi attracteurs par effet inductif que les groupes alcoyles;

- $R_4$ est choisi parmi l'hydrogène, les chaînes hydrocarbonées les halogènes les groupes électro-attracteurs de préférence par effet inducteur mésomère;

- $R_5$ est indifférent mais il est avantageux que il soit choisi parmi l'hydrogène, les groupes au moins aussi attracteurs par effet inductif que les groupes alcoyles ;

- $R_6$ choisi parmi les chaînes hydrocarbonées les halogènes les groupes électroattracteurs par effet inducteur mais non mésomère.

Dans un premier temps, s'il en reste de libre, les positions en ortho et para sont halogénées par l'agent d'halogénation.

Dans un deuxième temps, si on limite les quantités à la stoechiométrie (ou à un excès d'environ 10 % par rapport à la stoechiométrie pour une seule déalcoylation) mise en jeux de l'agent d'halogénation, un des groupes alcoyles et si l'on ne limite pas les deux groupes alcoyles sont monohalogénés en position $\alpha$ pour donner des composés qu'il a été possible d'isoler et qui sont de structure:

$$Ar\text{-}N(\text{-}CH_{2\text{-}x}Z_x\text{-}R_1')(\text{-}CH_{2\text{-}y}Z_y\text{-}R_2') \qquad \text{(III')}$$

avec Z représentant l'Halogène apporté par l'agent Halogénant;

$R_1$' étant un radical alcoyle d'au plus 4 carbones, de préférence un hydrogène et

$R_2$' étant un radical alcoyle d'au plus 4 carbones, de préférence un hydrogène.

$R_1$' et $R_2$' pouvant être reliés pour ne faire qu'un seul radical formant cycle avec l'azote.

On reviendra sur ce type de composé au cours de la description.

Cette déalcoylation est particulièrement surprenante quant à sa sélectivité puisque les positions méta ne sont pas touchées et que les carbones des chaînes alcoyles, situés en $\alpha$ de la fonction aniline ne sont pas touchés sensiblement plus d'une fois ce qui permet un économie de réactifs coûteux et réduit les quantités d'effluents, il est même possible de sélectivement mono déalcoyler l'aniline (lato sensu).

Au cours de l'étude qui a mené à la présente invention il a été montré que cette halogénation ne porte que sur les carbones des chaînes alcoyles liés directement à la fonction aniline et sur les positions en ortho et en para de la fonction aniline si elles sont libres.

En général, cette étape d'halogénation a lieu à une température comprise entre 0 et 100°C de préférence aux alentours entre 0°C et 50°C dans des solvants chlorés ou simplement polaires, lorsque l'on utilise des solvants dont le point d'ébullition est inférieur à 100°C, il est pratique de travailler au reflux, le cas échéant sous pression réduite pour amener la température de reflux dans la zone préférée.

En général le procédé comporte l'étape suivante:

c) hydrolyse du produit de l'étape b) d'halogénation. L'hydrolyse est connue en elle-même et est menée de préférence en présence d'absorbeur des acides dégagés.

L'halogénation peut introduire dans la molécule divers halogènes choisis parmi le chlore le brome ou l'iode.
En général pour des raisons économique on préfère le chlore.

Toutefois lorsque l'on désire apporter un halogène spécifique sur le noyau il convient d'utiliser un agent d'halogénation apportant cet halogène, il en va de même lorsque l'on désire éviter une perhalogénation des positions libres ortho et para par rapport à l'aniline: dans ce cas l'on halogène par un agent introduisant un halogène de rang supérieur à ceux existant dans le noyaux, puis on deshydrogénohalogène par de moyen connus en eux même pour être sélectif. On choisit comme solvant de cette halogénation des solvants inertes dans les conditions de l'expérience dont on peut citer comme paradigmes, des composés tels que le tétrachlorure de carbone, le chlorobenzène, le dichlorobenzène et l'acétonitrile, le plus souvent, l'agent d'halogénation est l'halogène moléculaire ou atomique.

Outre les halogènes eux mêmes, on peut citer comme agent d'halogénation, les halogénures de thionyle ou de sulfuryle $SO_nCL_2$ (avec n égale à 1 ou 2), le pentachlorure de phosphore $PCL_5$ , le mélange $PCL_3/CL_2$ les hypochlorites d'alcoyles et les composés de type $X_20$ (avec X représentant un halogène; sauf fluor et iode).

Lorsque ces réactifs ne sont pas naturellement des réactif radicalaire (comme c'est le cas pour les halogénures de thionyle ou de sulfuryle) sont utilisés en présence d'agent ou de conditions favorisant la formation de radicaux libre et notamment sous l'action de rayonnement actinique connu en eux même pour favoriser la formation des radicaux Hal•.

Le choix de la stoechiométrie permet de choisir entre une Halogénation du noyau, une monodéalcoylation ou une dialcoylation.

Dans le cas du brome, il convient de citer, lorsqu'ils sont stables et pas trop toxiques, les composés correspondants aux chlorés ci-dessus ainsi que les composés du type N Bromo Succinimide (NBS).

Dans le cas de l'iode le meilleur agent iodant reste l'iode moléculaire.

Ainsi les dérivés obtenus à partir de dichloro 1,2 trifluorométhyl 4 benzène ou de paratrifluorométhylchlorobenzène peuvent être transformés en trifluorométhyl 4 chloro 2 halogéno 6 aniline, par une halogénation en elle même connue, suivie d'une hydrolyse.

De manière inattendue les produits intermédiaires de formule

$$Ar\text{-}N(\text{-}CH_{2\text{-}x}Cl_x\text{-}R_1')(\text{-}CH_{2\text{-}y}Cl_y\text{-}R_2') \qquad (III)$$

où x = y = 1 ,

où Ar représente le radical issu de la formule (I) par suppression du radical $R_1$ en laissant une valence libre et chloration des positions ortho et méta éventuellement laissées libres;

sont suffisamment stables pour pouvoir être isolés. et constituer des précurseurs de nombreux dérivés chimiques.

En outre leur isolement du milieu réactionnel permet d'obtenir une meilleure pureté de l'aniline totalement ou partiellement déalcoylée, la séparation est très facilitée par le fait que les composés où y = 1 et x = 1 sont très majoritaires.

Le procédé marche bien surtout pour les anilines mono- ou dialcoylées (lato sensu) dont l'acide conjugué présente un pKa au plus égal à 2, avantageusement à 1, de préférence à 0.

Au cours de l'étude qui a mené à la présente invention il a également été montré qu'il était possible de déalcoyler sélectivement les mono- et les di-alcoylanilines selon la présente invention sans toucher le radical aromatique initial.

Cette technique consiste à mettre en oeuvre un procédé qui comporte l'étape suivante:

b') faire réagir la dite aniline avec de l'ammoniac ou une amine, avantageusement au plus secondaire, sous forme libre ou d'un de ses sels, en présence d'une quantité qui peut être catalytique de sel(s) de pyridine, avantageusement de halohydrate(s) de pyridine.

Cette surprenante réaction présente l'avantage pour les dialcoylanilines de pouvoir être rendue sélective et d'être possible même quand les alcoylanilines sont secondaires.

Le terme "aniline" doit être pris dans son acception la plus large c'est à dire qu'il désigne toute amine liée directement à un noyau aromatique (de préférence à un seul) appauvri en électron, cet appauvrissement est mesuré par le pKa de l'acide associé à la fonction aniline que l'on désire déalcoyler.

Ainsi par aniline, il convient de comprendre non seulement l'aniline proprement dite, mais encore les composés chimiques provenant de la substitution de l'aniline y compris les anilines accolées à d'autre(s) noyau(x) tel(s) que par exemple les naphtylamines, l'appauvrissement du noyau en électron peut être du à toute cause connue tel que la présence de substituant(s) électroattracteur(s) de toute nature ou la présence dans un des cycles d'un hétéroatome (noyau à 6 chaînons).

Bien entendu par amine, il convient aussi de comprendre le mélange d'amines telles que définies ci-dessus; l'amine peut être une pyridine.

Pyridine doit ici être entendue "lato sensu", c'est à dire comme toute molécule présentant un noyau pyridine dont l'atome d'azote ne possède pas de substituant.

Ainsi par pyridine, il convient de comprendre non seulement la pyridine proprement dite, mais encore les composés chimiques provenant de la substitution de la pyridine y compris les pyridines accolées à d'autre(s) noyau(x) tel(s) que par exemple la quinoléine. Il est préférable que les substituts de la pyridine proprement dite présente un point d'ébullition au plus égal à environ 200°C. Bien entendu par pyridine, il convient aussi de comprendre le mélange de pyridines telles que définies ci-dessus

Lorsque l'on désire déméthyler des diméthylanilines il est également possible de faire une mono ou une di-déalcoylation. Il y a mono déalcoylation lorsque l'acide correspondant au sel de pyridine et/ou au sel de l'amine est un acide relativement faible c'est à dire dont le pKa est au plus égal à environ 1, de préférence 3.

En général on utilise le chlorhydrate de pyridine, seul ou en présence de chlorhydrate d'amine.

Avantageusement, l'amine présente un pKa supérieur à celui de la pyridine de préférence d'au moins deux unités et plus préférentiellement de deux unité et demi.

Avantageusement, l'étape b') est menée à une température comprise entre 150 et 250°C, de préférence entre 180

et 220°C. Il convient de travailler avec un excès d'amine, de préférence avec un excès de chlorhydrate de pyridine, entre 1 et 50 équivalent, de préférence entre 2 et 10 (par rapport à la stoechiométrie de la déméthylation).

Un des aspects les plus surprenants et les plus intéressants de cette technique est que l'on ne constate aucune ammonolyse même dans le cas où l'aniline possède une fonction perfluoroalcoyle, en général trifluorométhyle.

La réaction est plus aisée lorsque le pKa de l'acide associé à l'aniline est au plus égal à 5, avantageusement à 2, de préférence à 0.

Les exemples, non limitatifs, suivants illustrent l'invention.

## Exemples types

$CF_3$-Φ'-CE → $CF_3$-F--N<

Dans un autoclave chemisé Téflon (de 100ml), on introduit dans l'ordre du p.chloro trifluorométhylbenzéne (1,29 g, 7,15 millimoles) du DMF (4g) et de l'amidure de sodium (0,56g, 14,3mol).

L'autoclave est fermé et on chauffe sous agitation à 180°C pendant 24 h. Après traitement du milieu réactionnel, on obtient le para-trifluorométhyl N,N-diméthylaniline. (TT 74%, RT 77%).

* Influence du solvant:

$CF_3$-Φ'-X→ $CF_3$-Φ'-N<

X= Br ou de préférence Cl

Conditions : $CF_3$-Φ'-CE 0,322 g (1,79 mmol)
$NANH_2$ 0,14 g 53,6 mmol)
DMF 4 g

autoclave chemisé Téflon (100 ml)

| Solvants | Θ° (°c) | t (H) | RR | TT | RT |
|---|---|---|---|---|---|
| **Exemple N° 1** DMF | 210 | 24 | 57 | 82 | 70 |
| **Exemple N° 2** Diméthyl acétamide | 220 | 24 | 30 | 76 | 37 |
| **Exemple N° 3** Tétraméthyl urée | 200 | 24 | 32 | 65 | 50 |
| **Exemple N° 4** Tétraméthyl guanidine | 21 | 24 | 30 | 96 | 32 |

* Plage de température: 150 - 230°C avec le DMF.

*

Cl NaOH Cl
$CF_3$ --- Φ- Cl ------------------> $CF_3$ --- Φ---- N <
DMF

Dans un autoclave d'1 titre en Hastelloy, on charge du dichloro- 3,4 trifluorométhylbenzéne (47,1 g , 0,212 mole), du DMF (200 g) et de la soude en pastilles (25,8 g, 0,645 mole).++

On ferme l'autoclave et on chauffe sous agitation à 210°C pendant 3 heures La pression atteint 9 bar.

Après traitement du milieu réactionnel, on obtient la trifluorométhyl-4 chloro-2 N,N-diméthylaniline (TT 92%) (RT 93%).

| ***Paramètres** | **Exemple N° 5** | **Exemple N° 6** | **Exemple N° 7** | **Exemple N° 8** | **Exemple N° 9** |
|---|---|---|---|---|---|
| Dichloro 3,4 trifluorométhyl-benzéne | 0,215 | 0,225 | 0,225 | 0,222 | 0,224 |
| (mmol) | 1 | 1 | 1 | 1 | 1 |
| DMF (g) | 4 | 4 | 4 | 4 | 4 |
| éq<sup>ts</sup>(équivalents) | 55 | 55 | 55 | 55 | 55 |
| NaOH (g) | ------ | ------- | 0,044 | 0,048 | ------ |
| éq<sup>ts</sup> | ------ | ------- | 1,1 | 1,2 | ------ |
| $H_2O$ (g) | 0,210 | 0,211 | ------ | ------ | ------ |
| éq<sup>ts</sup>(équivalents) | 12 | 12 | ------ | ------ | ------ |
| NaOH 37% (g) | ------ | ------- | ------ | ------ | 0,202 |
| éq<sup>ts</sup> (équivalents) | ------ | ------- | ------ | ------ | 1,8 NaOH 7 H2O |
| θ(=°C) | 195 | 195 | 195 | 195 | 195 |
| T (heures) | 20 | 20 | 20 | 20 | 20 |
| TT (%) | 45 | 75 | 59 | 78 | 91 |
| RT (%) | 87 | 86 | 71 | 67 | 64 |

**Exemple N° 10**

Du chlorure de sulfuryle (9,5g, 70mmol) est ajouté à solution de chloro-2 trifluorométhyl-4 N,N-diméthyl aniline (14,25g, 63,8mmol) dans du tétrachlorure de carbone (90ml). l'essai a été mené en présence d'une forte lampe et en l'absence de lumière.

Après chauffage à 70°C pendant 2 heures, la solution est traitée par un excès de soude à 50% dans l'eau.

L'analyse HPLC de la phase organique montre les résultats suivants :

| **Conditions** | **dichloro-2,6 trifluorométhyl-4 N,N-diméthyl aniline** | **dichloro-2,6 trifluorométhyl-4 N-méthyl aniline** |
|---|---|---|
| En présence de lampe | 5-10% | 85 % |
| A la lumière du jour | 68 % | 17 % |
| Dans l'obscurité | 90 % | 0 % |

**Exemple N° 11**

Un mélange de dichloro-2,6 trifluorométhyl-4 N,N-diméthyl aniline (4,5g, 17,4mmol) et de dichloro-2,6 trifluorométhyl-4 N-méthyl aniline (1,68g, 6,9mmol) en solution dans du tétrachlorure de carbone (60ml) est traité par du chlorure de sulfuryle (5,7g, 42,2mmol).

Le mélange réactionnel refroidi à 10°C est illuminé par l'intermédiaire d'une lampe UV pendant 6 heures, puis traité par un excès de soude à 50% dans l'eau.

L'analyse CLHP de la phase organique montre la présence de dichloro-2,6 trifluorométhyl-4 aniline (70% molaire).

Exemple N° 12

De la dichloro-2,6 trilfluorométhyl-4 N-méthyl aniline (1,318g, 5,4mmol) en solution dans du tétrachlorure de carbone (40ml) est traitée par du chlorure de sulfuryle (4,5g, 33mmol). Le mélange réactionnel refroidi à 10°C est illuminé par l'intermédiaire d'une lampe pendant une heure, puis traité par un excès de soude à 50% dans l'eau. L'analyse CLHP de la phase organique montre la présence de la dichloro-2,6 trifluorométhyl-4 aniline (83% molaire).

Exemple N° 13

Une quantité théorique de chlore gazeux est introduite dans une solution de dichloro-2,6 trifluorométhyl-4 N,N-diméthyl aniline (3g, 11,6mmol) en solution dans du tétrachlorure de carbone (60ml).
Pendant l'introduction de chlore, le mélange réactionnel est refroidi à 10°C et illuminé par l'intermédiaire d'une lampe UV. L'analyse CPHL de la phase organique après traitement, après 1 heure montre une conversion de 92% et un rendement en dichloro-2,6 trifluorométhyl-4 aniline de 64%.

Exemple N° 14

Une quantité théorique de chlore gazeux est introduite dans une solution de dichloro-2,6 trifluorométhyl-4 N,N-diméthyl aniline (2,25g, 8,7mmol) en solution dans du dichloro-1,2 benzène (65ml).
Pendant l'introduction de chlore, le mélange réactionnel est refroidi à 10°C et illuminé par l'intermédiaire d'une lampe UV.
L'analyse CLHP de la phase organique après traitement au bout de 50 minutes montre une conversion de 99% et un rendement en dichloro-2,6 trifluorométhyl-4 aniline de 92%.

Exemple N° 15

Une quantité théorique de chlore gaz:eux est introduite dans une solution refroidie à 10°C de dichloro-2,6 trifluorométhyl-4 *N,N-diméthyl* aniline (8,45mmol) dans du dichloro1,2 benzène (65ml). Après illumination de la masse réactionnel par l'intermédiaire d'une lampe UV pendant 15 minutes, la réaction est poursuivie à l'abri de la lumière pendant 2 heures. L'analyse CLHP de la phase organique après traitement montre une conversion de 98% et un rendement en dicholoro-2,6 trifluorométhyl-4 aniline de 34%.

Exemple N° 16

Dans un autoclave chemisé Téflon (de 100ml), on introduit dans l'ordre du trichloro-1,2,3 benzène (0,963g), du DMF (4g) et de la soude (0,4g).
L'autoclave est fermé et on chauffe sous agitation à 220°C pendant 24 heures. La conversion est alors de 78%.

Après traitement du milieu réactionnel, 0,517g du mélange de dichloro N,N-diméthyl anilines est mis au contact de chlorhydrate de pyridine (5,70g).

Le mélange est chauffé à 185°C pendant 45 minutes.
L'analyse CLHP du mélange montre la présence de dichloro-2,3 aniline (95%) et de dichloro-2,6 aniline (5%).

**Exemple N° 17**

Dans un autoclave chemisé Téflon (de 100ml), on introduit dans l'ordre du trichloro-1,2,4 benzène (0,766g), du DMF (4g) et de la soude (0,4g).
L'autoclave est fermé et on chauffe sous agitation à 220°C pendant 24 heures.
La conversion est alors de 82%

Après traitement du milieu réactionnel, 0,223 g du mélange de chloro N,N-deméthyl anilines est mis au contact de chlorhydrate de pyridine (3,2g).

Le mélange est chauffé à 185°C pendant 60 minutes.

L'analyse CLHP du mélange montre la présence de dichloro-2,4 aniline (96%) et de dichloro-3,4 aniline (4%).

**Exemple N° 18**

Dans un autoclave chemisé Téflon (de 100ml), on introduit dans l'ordre du tétrachloro-1,2,4,5 benzène (0,919g), du DMF (4g) et de la soude (0,4g).
L'autoclave est fermé et on chauffe sous agitation à 220°C pendant 24 heures.

La conversion est alors de 100%.

Après traitement du milieu réactionnel, 0,50g du mélange de trichloro N,N-diméthyl anilines est mis au contact de chlorhydrate de pyridine (5,8g).
Le mélange est chauffé à 190°C pendant 60 minutes.
L'analyse CLHP montre la présence de trichloro-2,4,5 aniline (100%).

**Exemple N° 19**

Dans un autoclave chemisé Téflon (de 100ml), on introduit dans l'ordre du dichloro-2,6 bromobenzène (0,992g), du DMF (4g) et de la soude (0,4g).
L'autoclave est fermé et on chauffe sous agitation à 220°C pendant 24 heures.
La conversion est alors de 89%.

Après traitement du milieu réactionnel, 0,43g du mélange de chloro N,N-diméthyl anilines est mis au contact de chlorhydrate de pyridine (5,2g).
Le mélange est chauffé à 190°C pendant 60 minutes.
L'analyse CLHP du mélange montre la présence de dichloro-2,6 aniline (43%) et de bromo-2 chloro-3 aniline (57%).

**Exemple N° 20**

Du chlorhydrate de pyridine (4,35g, 37,7mmol) est ajouté à du chloro-2,6 trifluorométhyl-4 N,N-diméthyl aniline (0,86g, 3,3mmol).

Le mélange est chauffé à 185°C pendant 2 heures.
L'analyse CLHP montre alors la présence de dichloro-2,6 trifluorométhyl-4 aniline (76% molaire).

Exemple N° 21 monodéméthylation

Dans un autoclave chemisé Téflon (de 100ml), on introduit du dichloro-2,6 trifluorométhyl-4 N,N-diméthyl aniline (0,86g, 3,3mmol) et de l'acétate de pyridinium (5g).
L'autoclave est fermé et on chauffe à 190°C pendant 2 heures.
L'analyse CLHP montre la présence de dichloro-2,6 trifluorométhyl-4 N-méthyl aniline (rdt 9% molaire).

**Exemple N° 22 (comparatif)**

Un mélange de dichloro-2,6 trifluorométhyl-4 N,N-diméthyl aniline (0,810g) et de chlorhydrate de diméthylamine (3,5g) est chauffé 1 heure à 180°C.
L'analyse CLHP du mélange indique l'absence de conversion.

**Exemple N° 23**

Un mélange de dichloro-2,6 trifluorométhyl-4 N,N-diméthylamine (0,745g), du chlorhydrate de pyridine (34mg) et du chlorhydrate de diméthylamine (3,24g) est chauffé 1 heure à 180°C.
L'analyse CLHP du mélange montre un rendement(20 ù) en dichloro-2,6 trifluorométhyl-4 aniline trois fois supérieur à la théorie si la pyridine était seule active.

**Exemple N° 24 (technique usuelle)**

Une solution de dichloro-2,6 trifluorométhyl-4 N,N-diméthyl aniline (1g) dans l'acide bromhydrique aqueux (47%, 5g) est portée au reflux pendant 5 heures.
L'analyse CLHP du mélange montre une conversion de 89% et un rendement en dichloro-2,6 trifluorométhyl-4 aniline de 55%.

**Exemple N° 25 comparatif**

L'objectif est de démontrer la nécessité du caractére radicalaire.

Dans un réacteur téflon de 100 ml, on introduit du chloro-2 nitro-6 N,N-diméthyl aniline (0,802 g, 4 mmol) en solution dans du chloroforme (10 ml). A cette solution, on ajoute une solution de brome (0,64 g, 4 mmol) dans du chloroforme (20 ml). Le mélange est agité à température ambiante pendant 4 heures. L'analyse du mélange réactionnel ne montre que la seule présence du produit de départ.

(Technique d'analyse : chromato gaz et spectro de masse).

**Exemple N° 26**

On cherche à démontrer la présence nécessaire d'une base.

Technique sans base

Dans un autoclave chemisé Teflon (100 ml), on introduit dans l'ordre du dichloro-3,4 trifluorométhyl-4 benzène (4,62 g, 21,4 mmol) et du diméthylformamide (22 g, 14 équivalents molaires).
L'autoclave est fermé et on chauffe sous agitation pendant 5 Heures à 200°C.
L'analyse du milieu réactionnel par chromatographie en pahse gazeuse indique une conversion de 3 % pour un rendement en diméthylanilines de 2 %.

Technique avec base

Dans un autoclave chemisé Tefon (100 ml), on introduit dans l'ordre du dichloro-3,4 trifluorométhyl-4 benzène (4,62 g, 21,4 mmol), du diméthylformamide (22 g, 14 équivalents molaires).
L'autoclave est fermé et on chauffe sous agitation pendant 5 heures à 200°C.
L'analyse du milieu réactionel par chromatographie en phase gazeuse indique une conversion de 95 % pour un rendement en chloro-2 trifluorométhyl-4 diméthylaniline de 80 %.

**Exemple N° 27 de régénération des pyridiniums et procédé de recyclage**

Le chlorydrate de pyridine peut être facilement obtenu par des techniques connus de l'homme de l'art comme par exemple traitement de lapuridine avec une quantité stoechiométrique d'une solution d'acide chlorhydrique à 36 % dans l'eau puis distillation azéotropique de l'eau avec du toluène.

**Procédé:**

De la dichloro-2,6 trifluorométhyl-4 diméthyl aniline (1,72 g, 6,6 mmol) est chauffée à 180°C 1 heure en présence de chlorhydrate de pyridine (8,7 g, 37,7 mmol). Un dosage de la masse réactionnelle indique une conversion de 100%.
Le milieu réactionnel est alors refroidi à 140°C et on ajoute sous agitation de l'ortho-xylène (5ml). Au bout de 2 Minutes, l'agitation est arrêtée et on assiste à une séparation en deux couches. La phase supérieure est récupérée.
L'analyse par chromatographie en phase gazeuse montre la présence de dichloro-2,6 trifluorométhyl-4 aniline (1,47g, rendement 97 %).
La phase inférieure contenant un mélange de chlorydrates de méthylpyridinium et pyridinium est régénérée à 200°C par l'action de l'ammoniac. La pyridine récupérée par distillation peut être recyclée.

## Revendications

1. Procédé caractérisé par le fait qu'il comporte une étape a) consistant à faire réagir un composé de formule (I)

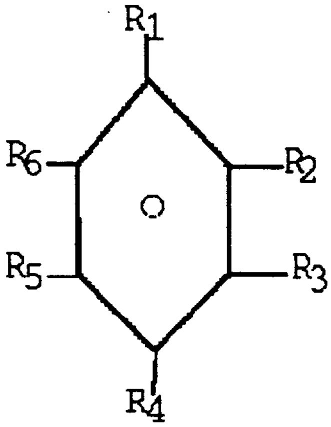

(I)

avec :

- $R_1$ choisi parmi les groupes dont les anions constituent d'excellents groupes partants,.,.avantageusement parmi les atomes de brome ou de chlore, de préférence ce dernier ; sont considérés comme bon groupes partant, ceux dont l'acide associé à l'anion présentent une valeur de pKa au plus égale à 1, avantageusement à 0, de préférence à -1
- $R_2$ choisi parmi l'hydrogène, les chaînes hydrocarbonées, les halogènes, les groupes électro-attracteurs (GEA) pu effet inducteur mais non mésomère ;
- $R_3$ est indifférent mais il est avantageux que il soit choisi parmi l'hydrogène, les groupes au moins aussi attracteurs par effet inductif que les groupes alcoyles ;
- $R_4$ est choisi parmi l'hydrogène, les chaînes hydrocarbonées les halogènes les groupes électro-attracteurs par effet inducteur mais non mésomère ;
- $R_5$ est indifférent mais il est avantageux que il soit choisi parmi l'hydrogène, les groupes au moins aussi attracteurs par effet inductif que les groupes alcoyles ;
- $R_6$ choisi parmi les chaînes hydrocarbonées les halogènes les groupes électroattracteurs par effet inducteur mais non mésomère ;

avec la condition que au moins un des groupes $R_{1,}$ $R_6$ et de préférence $R_4$ est électro- attracteur pu effet inductif ;

sur un dialcoylamide en présence d'une base et à une température comprise entre 150 et 300°C de préférence entre 180°C et 250°C *réaction au cours de laquelle le groupe partant $R_1$ est remplacé par une fonction* dialcoyl*aniline.*

**2.** Procédé selon la revendication 1, caractérisé par le fait que la réaction est menée en présence de cuivre. ce dernier est de préférence introduit sous forme de sel cuivreux

**3.** Procédé selon les revendications 1 et 2, caractérisé par le fait que le groupe électro-attracteur (GEA) est choisi parmi les groupes alcoxycarbonyle, halogène léger (Fluor, chlore, brome), le plus souvent chlore et de préférence perhalogénoalcoyle.

**4.** Procédé selon les revendications 1 à 3, caractérisé par le fait que les radicaux $R_3$ et $R_5$ sont avantageusement peu encombrants et de préférence des hydrogènes, des pseudos-halogènes ou des halogènes, avantageusement fluor ou chlore.

**5.** Procédé selon les revendications 1 à 4, caractérisé par le fait que Les amides sont des préférence les dialcoylamides des acides carboxyliques légers (au plus 10 atomes de carbone).

**6.** Procédé selon les revendications 1 à 5, caractérisé par le fait que les dialcoylamides sont des dialcoylformamides, les radicaux alcoyles greffés sur l'azote des amides étant avantageusement légers, de manières que le nombre d'atome de carbone n'excède 6, avantageusement 4, de préférence 3.

7. Procédé selon les revendications 1 à 6, caractérisé par le fait que dans la formule (I) $R_1$ est chlore ou brome de préférence chlore ; $R_2$ est fluor, chlore ou brome de préférence chlore ; $R_3$ est hydrogène, fluor, chlore ou brome de préférence chlore ; $R_4$ est perfluoroalcoyle, en général trifluorométhyle.

8. Procédé selon les revendications 1 à 7, caractérisé par le fait que il comporte en outre l'étape suivante :

   b) traitement de l'aniline obtenue en l'étape a) par halogénation en elle même connue au moyen d'un agent d'halogénation de type radicalaire.

9. Procédé selon la revendication 8, caractérisé par le fait que l'étape d'halogénation a lieu à une température comprise entre 0 et 100°C de préférence aux alentours entre 0°C et 50°C

10. Procédé selon les revendications 1 à 9, caractérisé par le fait que comme agent d'halogénation, le chlorure de thionyle $SO_2CL_2$, le pentachlorure de phosphore $PCL_5$,.,.le mélange $PCL_3CL_2$. les hypochlorites d'alcoyles et les composés de type $X_20$ (avec X représentant un halogène ; sauf fluor et iode)

11. Procédé selon les revendications 1 à 10, caractérisé par le fait que l'agent d'halogénation est l'halogéne moleculaire ou atomique (ou en d'autre terme l'halogène sous forme radicalaire)

12. Procédé selon les revendications 1 à 7, caractérisé par le fait qu'il comporte l'étape suivante ;

   b') faire réagir l'aniline obtenue en l'étape a) avec de l'ammoniac ou une amine, avantageusement au plus secondaire, sous forme libre ou d'un de ses sels, en présence d'une quantité qui peut être catalytique de sel(s) de pyridine, avantageusement de halohydrate(s) de pyridine.

13. Procédé selon la revendication 12, caractérisé par le fait que l'amine présente un pKa supérieur à celui de la pyridine de préférence d'au moins deux unités et plus préférentiellement de deux unités et demi

14. Procédé selon les revendications 12 et 13, caractérisé par le fait que l'étape b') est menée à une température comprise entre 150 et 250°C, de préférence entre 180 et 220°C

15. Produits intermédiaires de formule :

$$Ar\text{-}N(\text{-}CH_{2\text{-}x}Cl_x\text{-}R_1')(\text{-}CH_{2\text{-}y}Cl_y\text{-}R_2') \quad (III)$$

où x et y ont la valeur un.
où Ar représente le radical issu de la formule (I) par suppression du radical $R_1$ en laissant une valence libre et chloration des positions ortho et para éventuellement laissées libres;

## Claims

1. Process, characterized in that it entails a step a) that consists in reacting a compound of formula (I)

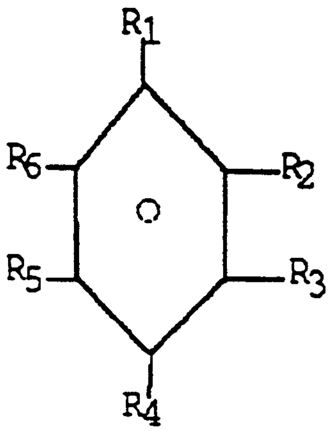

(I)

where:

- $R_1$ is chosen from groups whose anions constitute excellent leaving groups, advantageously from bromine or chlorine atoms, preferably the latter; good leaving groups are considered to be those for which the acid associated with the anion possesses a pKa value equal at most to 1, advantageously to 0 and preferably to -1;
- $R_2$ is chosen from hydrogen, hydrocarbon chains, halogens and groups which are electron-attracting (EAG) through an inductive but not a mesomeric effect;
- $R_3$ is immaterial in nature, but it is advantageous for it to be chosen from hydrogen and groups which exert at least as much attraction through an inductive effect as alkyl groups;
- $R_4$ is chosen from hydrogen, hydrocarbon chains, halogens and groups which are electron-attracting through an inductive but not a mesomeric effect;
- $R_5$ is immaterial in nature, but it is advantageous for it to be chosen from hydrogen and groups which exert at least as much attraction through an inductive effect as alkyl groups;
- $R_6$ is chosen from hydrocarbon chains, halogens and groups which are electron-attracting through an inductive but not a mesomeric effect; with the proviso that at least one of the groups $R_1$, $R_6$ and preferably $R_4$ is electron-attracting through an inductive effect;

  with a dialkylamide in the presence of a base and at a temperature of between 150 and 300°C, and preferably between 180 and 250°C, during which reaction the leaving group $R_1$ is replaced by a dialkylaniline functional group.

**2.** Process according to claim 1, characterized in that the reaction is carried out in the presence of copper; the latter is preferably introduced in the form of a cuprous salt.

**3.** Process according to claims 1 and 2, characterized in that the electron-attracting group (EAG) is chosen from alkoxycarbonyl, light halogen (fluorine, chlorine, bromine), most often chlorine and preferably perhaloalkyl groups.

**4.** Process according to claims 1 to 3, characterized in that the radicals $R_3$ and $R_5$ are advantageously relatively non-obstructing and preferably hydrogens, pseudohalogens or halogens, advanageously fluorine or chlorine.

**5.** Process according to claims 1 to 4, characterized in that the amides are preferably dialkylamides of light carboxylic acids (not more than 10 carbon atoms).

**6.** Process according to claims 1 to 5, characterized in that the dialkylamides are dialkylformamides, the alkyl radicals grafted on the nitrogen of the amides advantageously being light, so that the number of carbon atoms does not exceed 6, advantageously 4 and preferably 3.

**7.** Process according to claims 1 to 6, characterized in that, in the formula (I), $R_1$ is chlorine or bromine, preferably chlorine; $R_2$ is fluorine, chlorine or bromine, preferably chlorine; $R_3$ is hydrogen, fluorine, chlorine or bromine, preferably chlorine; $R_4$ is perfluoroalkyl, in general trifluoromethyl.

**8.** Process according to claims 1 to 7, characterized in that it additionally entails the following step:

b) treatment of the aniline obtained in step a) by halogenation, known per se, by means of a halogenating agent of the free-radical type.

**9.** Process according to claim 8, characterized in that the halogenation step takes place at a temperature of between 0 and 100°C, and preferably in the region of a point between 0°C and 50°C.

**10.** Process according to claims 1 to 9, characterized in that the halogenating agent is chosen from thionyl chloride $SO_2Cl_2$, phosphorus pentachoride $PCl_5$, a $PCl_3/Cl_2$ mixture, alkyl hypochlorites and compounds of the type $X_2O$ (with X representing a halogen; other than fluorine and iodine).

**11.** Process according to claims 1 to 10, characterized in that the halogenating agent is the molecular or atomic halogen (or, in other words, halogen in the free-radical form).

**12.** Process according to claims 1 to 7, characterized in that it entails the following step:

b') reacting the aniline obtained in step a) with ammonia or an amine which is advantageously at most secondary, in free form or the form of one of its salts, in the presence of an amount which my be catalytic of pyridine salt(s), advantageously of pyridine hydrohalide(s).

**13.** Process according to claim 12, characterized in that the amine possesses a pKa which is greater than that of pyridine, preferably by at least two units and more preferably by two and a half units.

**14.** Process according to claims 12 and 13, characterized in that step b') is carried out at a temperature of between 150 and 250°C, and preferably between 180 and 220°C.

**15.** Intermediate products of formula:

$$Ar\text{-}N(\text{-}CH_{2\text{-}x}Cl_x\text{-}R_1')(\text{-}CH_{2\text{-}y}Cl_y\text{-}R_2') \quad (III)$$

where x and y have the value one;
where Ar represents the radical derived from the formula (I) by elimination of the radical $R_1$, leaving a free valence, and chlorination of the ortho and meta positions left free where appropriate.

**Patentansprüche**

**1.** Verfahren, dadurch gekennzeichnet, daß es einen Verfahrensschritt a) umfaßt, bei dem man eine Verbindung der Formel (I)

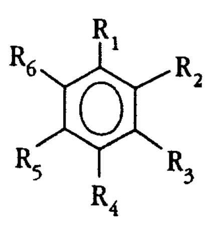

(I)

wobei:

- $R_1$ ausgewählt ist aus Gruppen, deren Anionen ausgezeichnete Abgangsgruppen darstellen, vorzugsweise aus Brom- oder Chloratomen, vorzugsweise aus letzteren; als gute Abgangsgruppen werden insbesondere solche Gruppen angesehen, deren mit dem Anion verbundene Säure einen $pK_a$-Wert von höchstens 1 aufweist, vorzugsweise von 0, insbesondere von -1;
- $R_2$ ausgewählt ist aus Wasserstoff, Kohlenwasserstoffketten, Halogenen sowie aufgrund eines Induktionseffektes, jedoch nicht eines Mesomerieeffektes elektronenziehenden Gruppen;

- $R_3$ beliebig ist, jedoch vorzugsweise ausgewählt ist aus Wasserstoff sowie Gruppen, die aufgrund eines Induktionseffektes mindestens genauso elektronenziehend sind wie Alkylgruppen;
- $R_4$ ausgewählt ist aus Wasserstoff, Kohlenwasserstoffketten, Halogenen sowie aufgrund eines Induktionseffektes, jedoch nicht aufgrund eines Mesomerieeffektes elektronenziehenden Gruppen;
- $R_5$ beliebig ist, jedoch vorzugsweise ausgewählt ist aus Wasserstoff sowie Gruppen, die aufgrund eines Induktionseffektes mindestens genauso elektronenziehend sind wie Alkylgruppen;
- $R_6$ ausgewählt ist aus Kohlenwasserstoffketten, Halogenen sowie Gruppen, die aufgrund eines Induktionseffektes, jedoch nicht aufgrund eines Mesomerieeffektes elektronenziehend sind;

jedoch mit der Maßgabe, daß mindestens eine der Gruppen $R_1$, $R_6$ und vorzugsweise $R_4$ aufgrund eines Induktionseffektes elektronenziehend ist,
mit einem Dialkylamid in Gegenwart einer Base und bei einer Temperatur zwischen 150 °C und 300 °C, vorzugsweise bei einer Temperatur zwischen 180 °C und 250 °C, reagieren läßt, wobei während der Reaktion die Abgangsgruppe $R_1$ durch eine Dialkylanilinfunktion ersetzt wird.

**2.** Verfahren nach Anspruch 1, dadurch gekennzeichnet, daß die Reaktion in Gegenwart von Kupfer ausgeführt wird, wobei letzteres vorzugsweise in Form eines Kupfer-I-salzes.

**3.** Verfahren nach Anspruch 1 oder 2, dadurch gekennzeichnet, daß die elektronenziehende Gruppe ausgewählt ist aus Alkoxycarbonylgruppen, leichtem Halogen (Fluor, Chlor, Brom), insbesondere Chlor, vorzugsweise Perhalogenalkyl.

**4.** Verfahren nach einem der Ansprüche 1 bis 3, dadurch gekennzeichnet, daß die Reste $R_3$ und $R_5$ insbesondere wenig sperrig sind und vorzugsweise Wasserstoff, Pseudohalogene oder Halogene darstellen, insbesondere Fluor oder Chlor.

**5.** Verfahren nach einem der Ansprüche 1 bis 4, dadurch gekennzeichnet, daß die Amide vorzugsweise Dialkylamide von leichten Carbonsäuren (mit höchstens 10 Kohlenstoffatomen) sind.

**6.** Verfahren nach einem der Ansprüche 1 bis 5, dadurch gekennzeichnet, daß die Dialkylamide Dialkylformamide sind, wobei die auf den Stickstoff der Amide gepfropften Alkylreste vorzugsweise leicht sind, so daß die Anzahl der Kohlenstoffatome 6, vorzugsweise 4, insbesondere 3, nicht übersteigt.

**7.** Verfahren nach einem der Ansprüche 1 bis 6, dadurch gekennzeichnet, daß in der Formel (I) der Rest $R_1$ Chlor oder Brom, vorzugsweise Chlor, ist; der Rest $R_2$ Fluor, Chlor oder Brom, vorzugsweise Chlor, ist; der Rest $R_3$ Wasserstoff, Fluor, Chlor oder Brom, vorzugsweise Chlor, ist; und der Rest $R_4$ Perfluoralkyl, im allgemeinen Trifluormethyl, ist.

**8.** Verfahren nach einem der Ansprüche 1 bis 7, dadurch gekennzeichnet, daß es außerdem den folgenden Verfahrensschritt umfaßt:

b) Behandlung des in Verfahrensschritt a) erhaltenen Anilins durch an sich bekannte Halogenierung mittels eines Halogenierungsmittels vom Radikaltyp.

**9.** Verfahren nach Anspruch 8, dadurch gekennzeichnet, daß der Verfahrensschritt der Halogenierung bei einer Temperatur zwischen 0 °C und 100 °C stattfindet, vorzugsweise bei etwa 0 °C bis 50 °C.

**10.** Verfahren nach einem der Ansprüche 1 bis 9, dadurch gekennzeichnet, daß als Halogenierungsmittel Thionylchlorid $SO_2Cl_2$, Phosphorpentachlorid $PCl_5$ oder eine Mischung aus $PCl_3/Cl_2$, Alkylhypochloriten und Verbindungen des Typs $X_2O$ (wobei X ein Halogen außer Fluor und Iod darstellt) verwendet wird.

**11.** Verfahren nach einem der Ansprüche 1 bis 10, dadurch gekennzeichnet, daß das Halogenierungsmittel ein Halogen in molekularer Form oder in atomarer Form (mit anderen Worten ein Halogen in Radikalform) ist.

**12.** Verfahren nach einem der Ansprüche 1 bis 7, dadurch gekennzeichnet, daß es den folgenden Verfahrensschritt umfaßt:

b') Reaktion des in Verfahrensschritt a) erhaltenen Anilins mit Ammoniak oder einem Amin, vorzugsweise einem höchstens sekundären Amin, entweder in freier Form oder in Form eines seiner Salze, in Gegenwart

eines oder mehrerer Pyridinsalze, vorzugsweise Pyridinhalohydrat(e), wobei diese Menge auch katalytisch sein kann.

**13.** Verfahren nach Anspruch 12, dadurch gekennzeichnet, daß das Amin einen $pK_a$-Wert aufweist, der größer ist als derjenige des Pyridins, vorzugsweise um mindestens zwei Einheiten und insbesondere um zweieinhalb Einheiten.

**14.** Verfahren nach einem der Ansprüche 12 oder 13, dadurch gekennzeichnet, daß der Verfahrensschritt b') bei einer Temperatur zwischen 150 °C und 250 °C, vorzugsweise bei einer Temperatur zwischen 180 °C und 220 °C, durchgeführt wird.

**15.** Zwischenprodukte der Formel (III)

$$Ar\text{-}N(\text{-}CH_{2\text{-}x}Cl_x\text{-}R_{1'})(\text{-}CH_{2\text{-}y}Cl_y\text{-}R_{2'}) \qquad (III)$$

wobei x und y den Wert eins besitzen und
wobei Ar dem Rest aus der Formel (I) entspricht, und zwar unter Weglassung des Restes $R_1$, wobei man eine Valenz frei läßt, und Chlorierung der gegebenenfalls frei gelassenen ortho- und para-Positionen.